# EUROPÄISCHE PATENTSCHRIFT

(11) **EP 0 374 122 B1**
(45) Veröffentlichungstag und Bekanntmachung des Hinweises auf die Patenterteilung: **07.09.1994**
(21) Anmeldenummer: 89890313.3
(22) Anmeldetag: 07.12.1989
(51) Int. Cl.: C12M 1/00, C12J 1/10, B01D 19/02

(54) **Verfahren und Vorrichtung zur Schaumführung bei einem Fermentationsprozess zur Essigherstellung**
Process and installation for dominating the foam in a fermentation process for making vinegar
Procédé et installation pour maîtriser la mousse au cours d'un procédé de fermentation destiné à la production de vinaigre

(30) Priorität: 13.12.1988 AT 3034/88
(43) Veröffentlichungstag der Anmeldung: 20.06.1990
(73) Patentinhaber: Heinrich Frings GmbH & Co. KG, D-53115 Bonn (DE)
(72) Erfinder: Wittler, Rüdiger, Dr., D-5308 Rheinbach (DE)
(74) Vertreter: Hübscher, Heiner, Dipl.-Ing.

(56) Entgegenhaltungen:
- EP-A- 0 026 527
- WO-A-88/04950
- AT-B- 206 866
- US-A- 4 752 564
- PROCESS BIOCHEMISTRY, Band 13, Nr. 11, November 1978; F. MÜLLER, Seiten 10-11#

## Beschreibung

Die Erfindung bezieht sich auf ein Verfahren zur Schaumführung bei einem Fermentationsprozeß zur Essigherstellung, wobei der auf der Oberfläche eines Fermentationssubstrates anfallende Schaum entlang einer Förderstrecke unter Einwirkung von Fliehkräften um die Förderstreckenachse in einen in Richtung der Förderstrecke abziehenden Gasanteil und einen sich radial abscheidenden, gegebenenfalls noch Schaumreste aufweisenden Flüssigkeitsanteil aufgetrennt wird.

Um den bei Fermentationsprozessen zur Essigherstellung anfallenden Schaum nicht vollständig in einen Gas- und einen Flüssigkeitsanteil auftrennen zu müssen, ist es bekannt (AT-B-206 866), aus dem Schaum in einem einen Umlaufkörper mit radialen Flügeln aufnehmenden Gehäuse einen Gasanteil abzutrennen, indem der axial in das Gehäuse eintretende Schaum durch die Flügel des Umlaufkörpers einer Fliehkraftwirkung ausgesetzt wird, die zu einem radialen Abscheiden des Flüssigkeitsanteiles führt, so daß der Hauptgasanteil aus dem Umlaufkörper axial flüssigkeits- und schaumfrei abgezogen werden kann. Der noch mit Schaumresten behaftete Flüssigkeitsanteil wird über einen Flüssigkeitsablauf im Bereich des Gehäusemantels ausgetragen und dem Fermentationssubstrat wieder zugeführt, wobei ein stauungsfreier Schaumkreislauf zwischen dem Fermenter und dem Umlaufkörper aufgebaut wird, um nicht eine vollständige, mit einem hohen Energiebedarf verbundene Auftrennung des Schaumes in einen Gas- und einen Flüssigkeitsanteil vornehmen zu müssen. Diese Kreislaufführung eines Schaumanteiles hat sich bei ordnungsgemäß ablaufenden Fermentationsprozessen zur Essigherstellung bewährt, kann aber in Störungsfällen zu einer übermäßigen Schaumausbildung führen, so daß der Antrieb für den Umlaufkörper überlastet wird und die Schaumauftrennung ausfällt, was in weiterer Folge zu einer Unterbrechung des Fermentationsprozesses führen kann. Solch ein übermäßiger Anfall an Schaum kann beispielsweise bei einer submersen Essiggärung durch eine Schädigung der Essigbakterien im Zusammenhang mit einer Störung der Sauerstoffversorgung, einem zu schnellen Wechsel der Konzentration an Alkohol und Essigsäure oder im Zusammenhang mit Bedienungsfehlern auftreten.

Der Erfindung liegt somit die Aufgabe zugrunde, ein Verfahren zur Schaumführung bei einem Fermentationsprozeß zur Essigherstellung der eingangs geschilderten Art so zu verbessern, daß die Schaumbelastung erheblich verringert werden kann, ohne den Energiebedarf für die Auftrennung des Schaumes in einen Gas- und einen Flüssigkeitsanteil vergrößern zu müssen.

Die Erfindung löst die gestellte Aufgabe dadurch, daß der beim Auftrennen des Schaumes anfallende Flüssigkeitsanteil mit den enthaltenden Schaumresten aus dem Fermentatlonsprozeß ausgeschieden wird.

Durch den Verzicht auf einen Schaumkreislauf und ein Ausscheiden des aus dem Schaum abgetrennten, gegebenenfalls noch mit Schaumresten beladenen Flüssigkeitsanteiles aus dem Fermentationsprozeß wird die Neigung zur Schaumbildung in überraschender Weise erheblich verringert, offensichtlich deshalb, weil die durch eine teilweise Lyse von geschädigten Bakterien entstehenden oberflächenaktiven Stoffe, die die Hauptursache für die Schaumbildung darstellen und mit dem Schaum dem Fermenter entnommen werden, nicht mehr über den abgeschiedenen Flüssigkeitsanteil in das Fermentationssubstrat gelangen und dort Anlaß für eine neue Schaumbildung sein können. Es tritt daher eine Beruhigung des Fermentationsprozesses mit einem erheblich verringerten Schaumanfall ein, der dem weiteren Fermentationsprozeß lediglich einen im Verhältnis zum Volumen des Fermentationssubtrates unwesentlich kleinen Flüssigkeitsanteil entzieht. Da mit dem Ausscheiden des beim Auftrennen des Schaumes anfallenden, schaumbelasteten Flüssigkeitsanteiles aus dem Fermentationsprozeß der Verstärkungseffekt, der sonst durch die wieder in das Fermentationssubstrat zurückgeführten schaumverursachenden Stoffe auftritt, unterbunden wird, bleibt auch der Schaumanfall in einem Störungsfall in zulässigen Grenzen. Außerdem ist aufgrund der verringerten Schaumbildung mit einem geringeren Energiebedarf für die Schaumauftrennung zu rechnen, so daß insgesamt ein höherer Wirkungsgrad erzielt werden kann.

Das Ausscheiden des anfallenden Schaumes aus einem Fermentationsprozeß ist zwar beispielsweise aus der Hefeproduktion bekannt, doch kann diese bekannte Schaumführung kein Vorbild für die Essigherstellung geben, weil im Gegensatz zur Essigherstellung die bei der Hefeproduktion am Fermentationsprozeß beteiligten Mikroorganismen das Endprodukt darstellen und daher auch aus dem anfallenden Schaum gewonnen werden können.

Der aus dem Fermentationsprozeß ausgeschiedene Flüssigkeits-und Schaumanteil kann verworfen oder unabhängig von dem aus dem Fermentationsprozeß ausgestoßenen Fertigprodukt weiterverarbeitet werden, beispielsweise in Form einer gesonderten Filtration. Besonders einfache Verhältnisse ergeben sich allerdings, wenn der aus dem Fermentationsprozeß ausgeschiedene Flüssigkeits- und Schaumanteil zur Weiterbehandlung dem ausgestoßenen Fertigprodukt zugemischt wird. Der aus dem zumindest zeitweise fortlaufend anfallenden Schaum gewonnene Flüssigkeits- und Schaumanteil wird vorzugsweise vor seiner Weiterverarbeitung gesammelt und zwischengelagert, was vor allem bei einem stoßweisen Anfall des Fertigproduktes empfehlenswert ist.

Zur Durchführung des Verfahrens kann von einer Vorrichtung mit einem drehbar in einem Gehäuse gelagerten Umlaufkörper, der radiale Flügel und zwischen den Flügeln axiale Durchtritte aufweist, mit einem an einem Fermenter angeschlossenen Schaumeinlauf auf der einen Stirnseite des Gehäuses, einem Gasabzug auf der anderen Stirnseite des Gehäuses und mit einem Flüssigkeitsablauf im Bereich des Gehäusemantels ausgegangen werden. Bei dieser Vorrichtung ist dann der Flüssigkeitsablauf mit einer aus dem Fermenter herausführenden Ablaufleitung zu verbinden. Wird der aus dem Fermenter ausgeschiedene Flüssigkeits- und Schaumanteil nicht verworfen, sondern weiterverarbeitet, so kann die Ablaufleitung mit einem gegenüber dem Fermenter abgeschlossenen Sammelbehälter verbunden werden, um den im Sammelbehälter anfallenden, gegebenenfalls noch mit Schaumresten behafteten Flüssigkeitsanteil dann beim Ausstoßen des Fertigproduktes aus dem Fermenter dem Fertigprodukt zur Weiterverarbeitung zumischen zu können.

Die vergleichsweise geringe Schaummenge macht es möglich, die Auftrennung des Schaumes in einen Gas- und einen Flüssigkeitsanteil zu verbessern, ohne den Energiebedarf hiefür zu vergrößern. Zu diesem Zweck kann der Umlaufkörper aus einem mittels der radialen Flügel auf einer Nabe abgestützten Umlaufzylinder bestehen, der in einem jeweils entgegen der Umlaufrichtung an die Flügel anschließenden Wandbereich radiale Durchtrittslöcher aufweist. Zufolge dieser Maßnahme wird der in den Umlaufzylinder axial eintretende Schaum nach seiner Erfassung durch die Flügel nicht unmittelbar unter dem Einfluß der Fliehkräfte in das den Umlaufkörper umschließende Gehäuse ausgeschleudert, sondern an der inneren Wand des Umlaufzylinders verdichtet, und zwar im Bereich des in Drehrichtung des Umlaufzylinders an den jeweiligen Flügel anschließenden Wandbereich, so daß der sich in diesem Wandbereich ansammelnde Flüssigkeits- und Schaumanteil sich erst in Drehrichtung entlang der inneren Wand des Umlaufkörpers bis zu dem in Drehrichtung folgenden Flügel ausbreiten muß, bevor er durch die radialen Durchtrittslöcher im Umlaufzylinder in das Gehäuse gelangen kann. Die damit verbundene verstärkte Fliehkrafteinwirkung unterstützt die Gasaustreibung und sichert damit die angestrebte Verbesserung hinsichtlich der Auftrennung in einen Gas- und einen Flüssigkeitsanteil.

Die durch das erfindungsgemäße Verfahren erreichbare Verringerung der anfallenden Schaummenge führt naturgemäß zu einer Verkleinerung der Vorrichtung zum Auftrennen des Schaumes in einen Gas- und einen Flüssigkeitsanteil. Dieser Umstand erlaubt es insbesondere bei kleineren Fermentern, die Vorrichtung zum Auftrennen des Schaumes nicht in herkömmlicher Weise mit horizontaler Drehachse anzuordnen, sondern den Umlaufkörper mit vertikaler Achse auf dem Fermenter zu lagern, wobei der Schaumeinlauf aus einer axialen, unteren Gehäuseöffnung des stirnseitig in den Fermenter ragenden Gehäuses besteht, so daß besonders vorteilhafte Konstruktionsbedingungen eingehalten werden können, die eine weitere Vereinfachung zulassen, wenn der Umlaufkörper fliegend gelagert wird.

Zum Abführen der abgetrennten Flüssigkeit kann aber auch von einem drehbar gelagerten Umlaufkörper, der radiale Flügel und zwischen den Flügeln axiale Durchtritte aufweist, einem am Fermenter angeschlossenen Schaumeinlauf auf der einen Stirnseite des Umlaufkörpers, einem Gasabzug auf der anderen Stirnseite des Umlaufkörpers und von einem an den Umlaufkörper anschließenden Flüssigkeitsablauf ausgegangen werden, wobei der Umlaufkörper aus einem mittels der radialen Flügel auf einer Nabe abgestützten Umlaufzylinder besteht, an den die aus dem Fermenter geführte Ablaufleitung des Flüssigkeitsablaufes mit einer entgegen der Drehrichtung des Umlaufkörpers gerichteten Einlaufmündung anschließt. Durch diese Maßnahme wird die sich auf dem Umlaufzylinder sammelnde, mit dem Umlaufkörper umlaufende Flüssigkeit in die gegenüber dem Umlaufkörper feststehende Ablaufleitung gedrückt und durch die Ablaufleitung aus dem Fermenter gefördert. Der Umlaufkörper muß zu diesem Zweck gegenüber der Ablaufleitung frei drehbar sein, was beispielsweise dadurch sichergestellt werden kann, daß der Umlaufzylinder zumindest auf einer Stirnseite über die Flügel hinaus axial vorragt und daß die Ablaufleitung des Flüssigkeitsablaufes mit ihrer Einlaufmündung an die Innenwandung des über die Flügel vorragenden Abschnittes des Umlaufzylinders anschließt. Eine andere Möglichkeit besteht darin, den Umlaufkörper an seiner Außenseite mit wenigstens einer mit dem Innenraum des Umlaufzylinders durch Durchtrittsöffnungen verbundene, ringförmige Sammelrinne zu versehen, an deren Außenwandung die Einlaufmündung der Ablaufleitung des Flüssigkeitsablaufes innenseitig anschließt. Die sich auf der Innenseite des Umlaufzylinders sammelnde Flüssigkeit wird in diesem Fall aufgrund der wirksamen Fliehkräfte durch die Durchtrittsöffnungen im Umlaufzylinder in die den Umlaufkörper umschließende Sammelrinne gedrückt, wo sie wiederum von der Ablaufleitung aufgenommen wird.

Besonders vorteilhafte Bedingungen hinsichtlich der Auftrennung des Schaumes in einen Gas- und einen Flüssigkeitsanteil ergeben sich dabei, wenn die ringförmige Sammelrinne im Bereich der Gasaustrittsseite an dem Umlaufkörper vorgesehen ist, weil im Bereich des Gasabzuges die Auftrennung des Schaumes in einen Gas- und einen Flüssigkeitsanteil im wesentlichen abgeschlossen ist.

Ein solcher Umlaufkörper kann zweckmäßig mit vertikaler Drehachse gelagert sein und mit seiner unteren, offenen Stirnseite den Schaumeinlauf bilden, so daß für den Schaumeinlauf keine gesonderten Konstruktionsmaßnahmen erforderlich werden. Die Rotationsenergie der Flüssigkeit ist dabei so groß, daß eine Druckförderung des abgetrennten Flüssigkeitsanteiles durch die nach oben aus dem Fermenter geführte Ablaufleitung ohne weiteres möglich wird.

Da der Umlaufkörper nur bei einem entsprechenden Schaumanfall angetrieben werden muß, ist es zweckmäßig ein Rücklaufen der abgeschiedenen Flüssigkeit in den Fermenter aus der Ablaufleitung und dem Umlaufkörper beim Stillsetzen des Umlaufkörpers zu vermeiden. Ein solcher Rückfluß der abgeschiedenen Flüssigkeit in den Fermenter kann vorteilhaft durch eine Sammelrinne am unteren Ende des nach unten vorragenden Abschnittes des Umlaufzylinders verhindert werden, wenn die Ablaufleitung mit ihrer Einlaufmündung in diese Sammelrinne ragt.

An Hand der Zeichnung wird das erfindungsgemäße Verfahren näher erläutert. Es zeigen
- Fig. 1: einen Fermenter zur submersen Essiggärung mit einer erfindungsgemäßen Schaumführung in einem schematischen Vertikalschnitt,
- Fig. 2: eine erfindungsgemäße Vorrichtung zum Auftrennen des anfallenden Schaumes in einen Gas- und einen Flüssigkeitsanteil in einem schematischen Axialschnitt in einem größeren Maßstab,
- Fig. 3: einen Schnitt nach der Linie III-III der Fig. 2,
- Fig. 4: eine Vorrichtung zum Auftrennen des anfallenden Schaumes in einen Gas- und einen Flüssigkeitsanteil mit vertikaler Drehachse in einem schematischen Axialschnitt,
- Fig. 5: eine der Fig. 4 entsprechende Darstellung einer Vorrichtung zum Auftrennen des anfallenden Schaumes mit einer Flüssigkeitsentnahme aus einem Umlaufzylinder,
- Fig. 6: eine Konstruktionsvariante einer Vorrichtung nach Fig. 5 ebenfalls in einem schematischen Axialschnitt und
- Fig. 7: eine der Fig. 6 entsprechende Darstellung einer weiteren Konstruktionsvariante.

Der zur submersen Essiggärung eingesetzte Fermenter 1 ist in üblicher Weise mit einer im Bodenbereich angeordneten Belüftungseinrichtung 2 versehen, die an eine Luftansaugleitung 3 angeschlossen ist, über die die für die Sauerstoffversorgung der Essigbakterien benötigte Luftmenge über ein Filter 4, ein Dosierventil 5 und eine Durchflußmeßeinrichtung 6 angesaugt wird. Am Ende einer Gärperiode wird bei einem vorgegebenen Restgehalt an Alkohol ein Teil des Fertigproduktes aus dem Fermenter 1 über ein Ausstoßventil 7 und eine Ausstoßpumpe 8 abgeführt und die abgeführte Produktmenge durch frische Maische ersetzt, die von einem nicht gezeichneten Vorratsbehälter über eine Einstoßpumpe 9, ein Dosierventil 10, eine Durchflußmeßeinrichtung 11 und eine Einstoßleitung 12 dem Rotor der Belüftungseinrichtung 2 axial zugeführt wird, um eine gleichmäßige und innige Vermischung der eingestoßenen Maische mit dem Fermentationssubstrat unter gleichzeitiger Belüftung sicherzustellen.

Der sich bei der submersen Essiggärung bildende Schaum gelangt über einen Schaumeinlauf 13 in eine Vorrichtung 14 zum Auftrennen des anfallenden Schaumes in einen Gas- und einen gegebenenfalls noch mit Schaumresten behafteten Flüssigkeitsanteil. Diese Vorrichtung 14 besteht im wesentlichen aus einem in einem Gehäuse 15 drehbar gelagerten, über einen Motor 16 antreibbaren Umlaufkörper 17, der eine Nabe 18 mit sich über die Nabenlänge erstreckenden radialen Flügeln 19 aufweist. Der über den Schaumeinlauf 13 auf der einen Stirnseite axial in das Gehäuse 15 eintretende Schaum wird durch die radialen Flügel 19 erfaßt, wobei unter der auftretenden Fliehkraftwirkung eine Abtrennung des Flüssigkeitsanteiles von dem axial durch den Umlaufkörper 17 strömenden Hauptgasanteil erfolgt, der auf der gegenüberliegenden Stirnseite des Gehäuses 15 durch einen Gasabzug 20 abgeführt wird. Der radial abgeschiedene, gegebenenfalls noch mit Schaumresten behaftete Flüssigkeitsanteil wird in das Gehäuse 15 ausgeschleudert und über einen an den Gehäusemantel angeschlossenen, durch eine Ablaufleitung 21 gebildeten Flüssigkeitsablauf aus dem Fermenter 1 herausgeführt. Der Flüssigkeitsanteil kann zu einem Ausguß gefördert und verworfen oder in einem Sammelbehälter 22 gesammelt werden. Durch das Ausscheiden des mit Schaumresten behafteten Flüssigkeitsanteils des anfallenden Schaumes aus dem Fermentationsprozeß wird eine Schaumbildung zufolge der in diesem Flüssigkeitsanteil enthaltenen, oberflächenaktiven Stoffe vermieden, die durch eine teilweise Lyse der geschädigten Essigbakterien entstehen und das Schäumen verursachen, so daß die Essiggärung beruhigt wird und mit einer wesentlich verringerten Schaumbildung gerechnet werden kann. Diese verringerte Schaumbildung erlaubt es auch, den bei der Auftrennung des Schaumes anfallenden Flüssigkeistanteil ohne weiteres aus dem Gärungsprozeß auszuscheiden, weil dieser Flüssigkeitsanteil im Vergleich zum Fermenterinhalt ein vernachlässigbar kleines Volumen darstellt.

Der sich im Sammelbehälter 22 ansammelnde Flüssigkeitsanteil des Schaumes kann fallweise aus dem Sammelbehälter abgeführt werden, und zwar vorteilhaft beim Essigausstoß, um diesen angesammelten Flüssigkeitsanteil mit dem ausgestoßenen Essig weiterzuverarbeiten. Zu diesem Zweck ist der Sammelbehälter 22 mit einer Auslaßleitung 23 über ein Steuerventil 24 an die Ausstoßpumpe 8 angeschlossen. Er kann aber auch über das Steuerventil 24 und die Ausstoßpumpe 8 bei geschlossenem Ausstoßventil 7 gesondert von dem Fertigprodukt einer Weiterverarbeitung, beispielsweise einer Filtration, zugeführt werden.

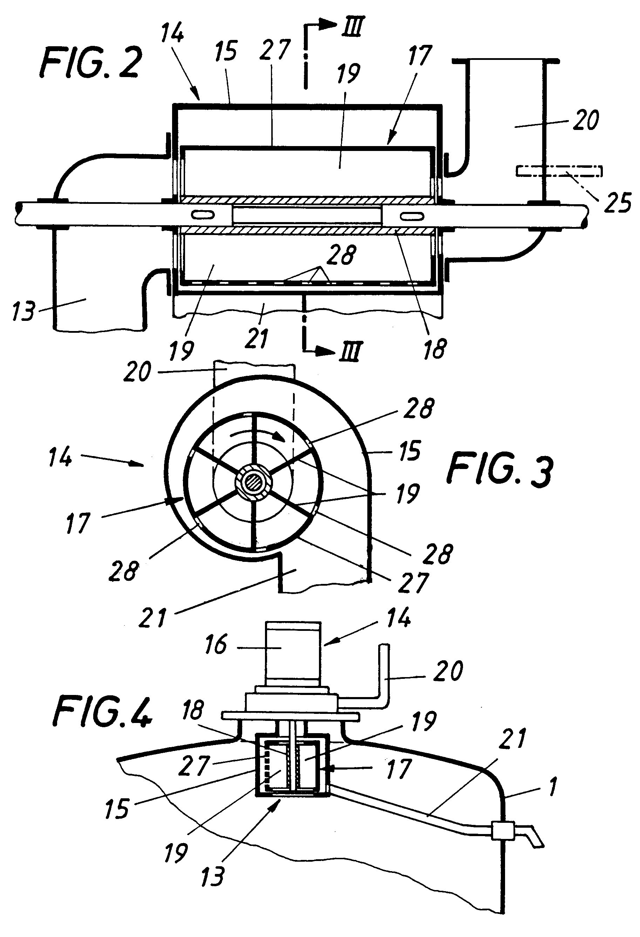

Aufgrund der geringen Schaumbildung wird die Vorrichtung 14 nur bei einem entsprechenden Schaumanfall eingeschaltet werden. Über einen Schaumfühler 25 kann die im Fermenter anstehende Schaumhöhe erfaßt und der Motor 16 in Abhängigkeit von der erfaßten Schaumhöhe über eine Steuereinrichtung 26 betätigt werden. Um ein unerwünschtes Einschalten des Motors 16 durch sich im Bereich des Schaumfühlers 25 ansammelnde Schaumreste 25 auszuschließen, kann der Schaumfühler auch im Gasabzug 20 unmittelbar nach dem Gasaustritt aus dem Umlaufkörper 17 angeordnet werden, wie dies in Fig. 2 strichpunktiert angedeutet ist. In diesem Fall ergeben sich besonders einfache Konstruktionsverhältnisse, wenn der Motor 16 nach dem Ansprechen des Schaumfühlers 25 über ein Zeitglied nur für eine vorgebbare Zeit eingeschaltet wird, so daß die Vorrichtung 14 verläßlich abgeschaltet bleibt, wenn sich der Gärungsverlauf beruhigt hat und kaum mehr Schaum anfällt.

Der geringe Schaumanfall macht es außerdem möglich, für eine bessere Auftrennung des Schaumes zu sorgen, indem der Umlaufkörper 17 mit einem von den Flügeln 19 getragenen Umlaufzylinder 27 versehen wird, der die axialen Schaumdurchlässe zwischen den radialen Flügen 19 segmentförmig abschließt. Dieser Umlaufzylinder 27 weist radiale Durchtrittslöcher 28 für den Flüssigkeitsanteil auf, die lediglich in einem entgegen der Drehrichtung des Umlaufkörpers 17 an die Flügel 19 anschließenden Wandbereich des Umlaufzylinders vorgesehen sind, so daß sich der radial abscheidende Schaum-und Flüssigkeitsanteil zunächst im Bereich der in Drehrichtung an die Flügel 19 anschließenden Wand des Umlaufzylinders 27 ansammelt, bevor er sich in Drehrichtung bis zu den Durchtrittslöchern 28 vor dem in Drehrichtung folgenden Flügel 19 ausbreitet und in das Gehäuse 15 ausgeschleudert werden kann. Damit wird ein zusätzlicher Gasanteil aus dem Flüssigkeitsanteil ausgeschieden, was zu einem verringerten Bedarf an Aufnahmevolumen für den Sammelbehälter 22 führt.

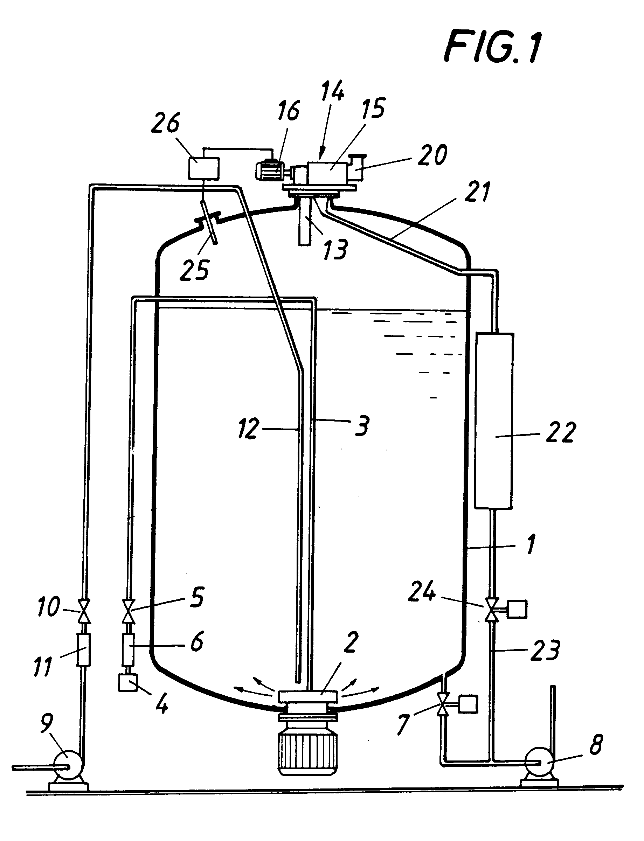

In den Fig. 1 bis 3 ist die Vorrichtung 14 zum Auftrennen des anfallenden Schaumes in einen Flüssigkeits- und einen Gasanteil in üblicher Weise mit horizontaler Drehachse auf dem Fermenter 1 angeordnet. Der zufolge des verringerten Schaumanfalles verminderte Energiebedarf für die Schaumauftrennung eröffnet, insbesondere bei kleineren Fermentern, die Möglichkeit, den Umlaufkörper 17 auch mit vertikaler Achse auf dem Fermenter 1 zu lagern, wie dies in der Fig. 4 angedeutet ist. In diesem Fall wird der Schaumeinlauf 13 durch eine untere axiale Gehäuseöffnung des stirnseitig in den Fermenter ragenden Gehäuses gebildet, wobei die Welle des Umlaufkörpers 17 vorteilhaft fliegend gelagert ist, was einen von der Anordnung eines Wellenlagers ungestörten Schaumeinlauf gewährleistet.

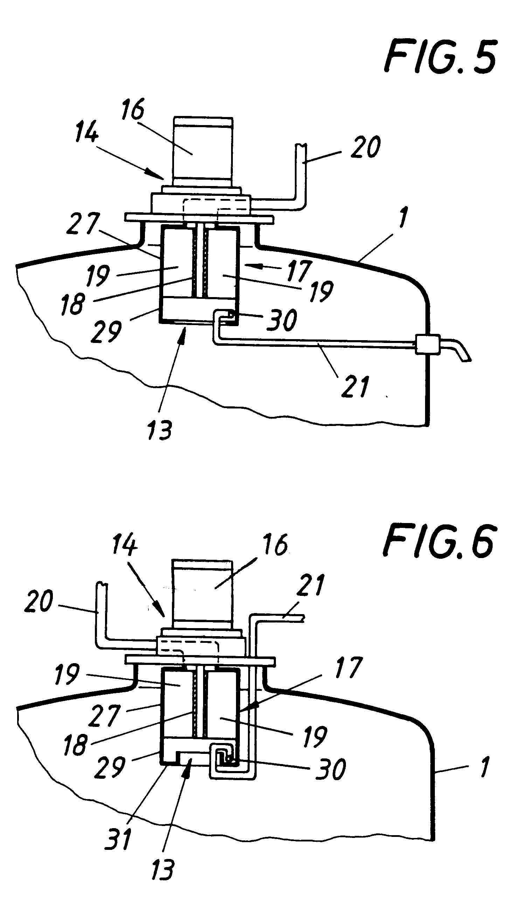

Die Vorrichtung 14 zum Auftrennen des anfallenden Schaumes in einen Flüssigkeits- und einen Gasanteil kann gemäß den Fig. 5 und 6 auch aus einem Umlaufkörper 17 mit einem geschlossenen Umlaufzylinder 27 bestehen, der bei einer Lagerung mit vertikaler Drehachse nach unten über die radialen Flügel 19 hinaus verlängert ist und einen vorragenden Abschnitt 29 bildet, der zugleich den Schaumeinlauf 13 darstellt. Fliehkraftbedingt sammelt sich bei diesem Umlaufkörper 17 der vom Schaum abgetrennte Flüssigkeitsanteil auf der Innenwandung des Umlaufzylinders 27, so daß dieser Flüssigkeitsanteil im Bereich des vorragenden Axialabschnittes 29 über die Ablaufleitung 21 abgenommen werden kann, die zu diesem Zweck eine gegen die Umlaufrichtung des Umlaufkörpers 17 gerichtete, an die Innenwandung des Abschnittes 29 anschließende Einlaufmündung 30 aufweist. Aufgrund der Rotationsenergie der sich am Umlaufzylinder 27 ansammelnden Flüssigkeit wird diese in die Ablaufleitung 21 unter einer entsprechenden Förderwirkung hineingedrückt, so daß eine Ableitung der abgetrennten Flüssigkeit auch nach oben möglich ist, wie dies in Fig. 6 dargestellt ist, in der die Ablaufleitung 21 nach oben durch den die Vorrichtung 14 tragenden Deckel geführt wird. Um in diesem Fall einen Rückfluß der von der Ablaufleitung 21 aufgenommenen Flüssigkeit in den Fermenter 1 beim Stillsetzen des Umlaufkörpers 17 zu vermeiden, bildet der nach unten vorragende Abschnitt 29 des Umlaufzylinders 27 eine Sammelrinne 31, in die die Ablaufleitung 21 mit ihrer Einlaufmündung 30 ragt.

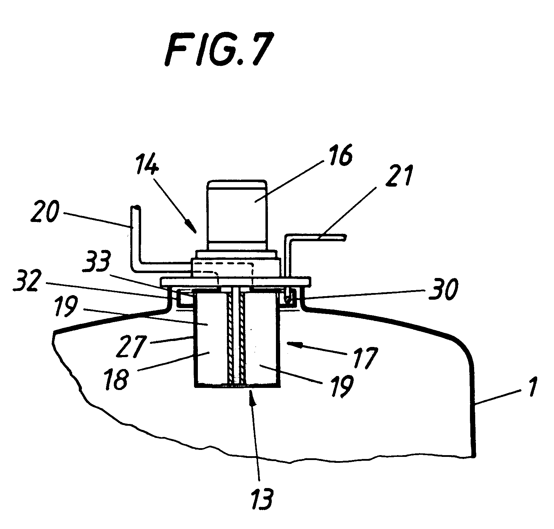

Die Vorrichtung 14 nach der Fig. 7 unterscheidet sich von der Vorrichtung nach der Fig. 6 im wesentlichen dadurch, daß der Umlaufkörper 17 an seiner Außenseite eine ringförmige Sammelrinne 32 trägt, die über Durchtrittsöffnungen 33 mit dem Innenraum des Umlaufzylinders 27 verbunden ist, so daß sich die aus dem Fermenter 1 abzuführende Flüssigkeit nicht innerhalb des Umlaufzylinders 27, sondern in der ringförmigen Sammelrinne 32 sammelt, die vorzugsweise im Bereich der Gasausstrittsseite des Umlaufkörpers 17 angeordnet ist. Die Ablaufleitung 21 des Flüssigkeitsablaufes schließt in diesem Fall mit ihrer Einlaufmündung 30 an die Außenwand der Sammelrinne 32 an, und zwar auf deren Innenseite.

## Patentansprüche

1. Verfahren zur Schaumführung bei einem Fermentationsprozeß zur Essigherstellung, wobei der auf der Oberfläche eines Fermentationssubtrates anfallende Schaum entlang einer Förderstrecke unter Einwirkung von Fliehkräften um die Förderstreckenachse in einen in Richtung der Förderstrecke abziehenden Gasanteil und einen sich radial abscheidenden, gegebenenfalls noch Schaumreste aufweisenden Flüssigkeitsanteil aufgetrennt wird, dadurch gekennzeichnet, daß der beim Auftrennen des Schaumes anfallende Flüssigkeitsanteil mit den enthaltenen Schaumresten aus dem Fermentations prozeß ausgeschieden wird.

2. Verfahren nach Anspruch 1, dadurch gekennzeichnet, daß der aus dem Fermentationsprozeß ausgeschiedene Flüssigkeits- und Schaumanteil unabhängig von dem aus dem Fermentationsprozeß ausgestoßenen Fertigprodukt weiterverarbeitet wird.

3. Verfahren nach Anspruch 1 oder 2, dadurch gekennzeichnet, daß der aus dem Fermentationsprozeß ausgeschiedene Flüssigkeits- und Schaumanteil dem aus dem Fermentationsprozeß ausgestoßenen Fertigprodukt zugemischt wird.

4. Verfahren nach Anspruch 2 oder 3, dadurch gekennzeichnet, daß der aus dem Fermentationsprozeß ausgeschiedene Flüssigkeits- und Schaumanteil vor der Weiterbehandlung zwischengelagert wird.

5. Vorrichtung zur Durchführung des Verfahrens nach einem der Ansprüche 1 bis 4 mit einem drehbar in einem Gehäuse (15) gelagerten Umlaufkörper (17), der radiale Flügel (19) und zwischen den Flügeln (19) axiale Durchtritte aufweist, mit einem an einem Fermenter (1) angeschlossenen Schaumeinlauf (13) auf der einen Stirnseite des Gehäuses (15), einem Gasabzug (20) auf der anderen Stirnseite des Gehäuses (15) und mit einem Flüssigkeitsablauf im Bereich des Gehäusemantels, dadurch gekennzeichnet, daß der Flüssigkeitsablauf mit einer aus dem Fermenter (1) herausführenden Ablaufleitung (21) verbunden ist.

6. Vorrichtung nach Anspruch 5, dadurch gekennzeichnet, daß die Ablaufleitung (21) mit einem gegenüber dem Fermenter (1) abgeschlossenen Sammelbehälter (22) verbunden ist.

7. Vorrichtung nach Anspruch 5 oder 6, dadurch gekennzeichnet, daß der Umlaufkörper (17) aus einem mittels der radialen Flügel (19) auf einer Nabe (18) abgestützten Umlaufzylinder (27) besteht, der in einem jeweils entgegen der Umlaufrichtung an die Flügel (19) anschließenden Wandbereich radiale Durchtrittslöcher (28) aufweist.

8. Vorrichtung nach Anspruch 7, dadurch gekennzeichnet, daß der Umlaufkörper (17) mit vertikaler Achse auf dem Fermenter (1) gelagert ist und daß der Schaumeinlauf (13) aus einer axialen, unteren Gehäuseöffnung des stirnseitig in den Fermenter (1) ragenden Gehäuses (15) besteht.

9. Vorrichtung zur Durchführung des Verfahrens nach einem der Ansprüche 1 bis 4 mit einem drehbar gelagerten Umlaufkörper (17), der radiale Flügel (19) und zwischen den Flügeln (19) axiale Durchtritte aufweist, mit einem an einem Fermenter (1) angeschlossenen Schaumeinlauf (13) auf der einen Stirnseite des Umlaufkörpers (17), einem Gasabzug (20) auf der anderen Stirnseite des Umlaufkörpers (17) und mit einem an den Umlaufkörper (17) anschließenden Flüssigkeitsablauf, dadurch gekennzeichnet, daß der Umlaufkörper (17) aus einem mittels der radialen Flügel (19) auf einer Nabe (18) abgestützten Umlaufzylinder (27) besteht, an den die aus dem Fermenter (1) geführte Ablaufleitung (21) des Flüssigkeitsablaufes mit einer entgegen der Drehrichtung des Umlaufkörpers (17) gerichteten Einlaufmündung (30) anschließt.

10. Vorrichtung nach Anspruch 9, dadurch gekennzeichnet, daß der Umlaufzylinder (27) zumindest auf einer Stirnseite über die Flügel (19) hinaus axial vorragt und daß die Ablaufleitung (21) des Flüssigkeitsablaufes mit ihrer Einlaufmündung (30) an die Innenwandung des über die Flügel (19) vorragenden Abschnittes (29) des Umlaufzylinders (27) anschließt.

11. Vorrichtung nach Anspruch 9, dadurch gekennzeichnet, daß der Umlaufkörper (17) an seiner Außenseite wenigstens eine mit dem Innenraum des Umlaufzylinders (27) durch Durchtrittsöffnungen (33) verbundene, ringförmige Sammelrinne (32) aufweist, an deren Außenwandung die Einlaufmündung (30) der Ablaufleitung (21) des Flüssigkeitsablaufes innenseitig anschließt.

12. Vorrichtung nach Anspruch 11, dadurch gekennzeichnet, daß die ringförmige Sammelrinne (33) im Bereich der Gasaustrittsseite an dem Umlaufzylinder (27) vorgesehen ist.

13. Vorrichtung nach einem der Ansprüche 9 bis 12, dadurch gekennzeichnet, daß der Umlaufkörper (17) mit vertikaler Drehachse gelagert ist und mit seiner unteren, offenen Stirnseite den Schaumeinlauf (13) bildet.

14. Vorrichtung nach den Ansprüchen 10 und 13, dadurch gekennzeichnet, daß der nach unten vorragende Abschnitt (29) des Umlaufzylinders (27) eine Sammelrinne (31) bildet, in die die Ablaufleitung (21) mit ihrer Einlaufmündung (30) ragt.

15. Vorrichtung nach einem der Ansprüche 9 bis 14, dadurch gekennzeichnet, daß die Ablaufleitung (21) nach oben aus dem Fermenter (1) geführt ist.

## Claims

1. A method of controlling the foam in a fermentation process for vinegar production, wherein the foam accumulating on the surface of a fermentation substrate is separated, along a conveying zone subject to the action of centrifugal forces about the axis of the conveying zone, into a gas fraction which is discharged in the direction of the conveying zone, and a liquid fraction which separates radially and which may still contain foam residues, characterised in that the liquid fraction occurring during the separation of the foam is discharged from the fermentation process together with the foam residues it contains.

2. A method according to claim 1, characterised in that the liquid and foam fraction discharged from the fermentation process is processed further independently of the finished product issuing from the fermentation process.

3. A method according to claim 1 or 2, characterised in that the liquid and foam fraction discharged from the fermentation process is added to the finished product issuing from the fermentation process.

4. A method according to claim 2 or 3, characterised in that the liquid and foam fraction issuing from the fermentation process is intermediately stored before further treatment.

5. Apparatus for performing the method according to any one of claims 1 to 4, comprising a revolving member (17) mounted rotatably in a housing (15) and having radial blades (19) and axial passages between the blades (19), with a foam inlet (13) on one end face of the housing (15), said inlet (13) being connected to a fermenter (1), a gas outlet (20) on the other end face of the housing (15), and with a liquid outlet in the region of the housing casing, characterised in that the liquid outlet is connected to a discharge line (21) leading out of the fermenter (1).

6. Apparatus according to claim 5, characterised in that the discharge line (21) is connected to a reservoir (22) which is sealed off from the fermenter (1).

7. Apparatus according to claim 5 or 6, characterised in that the revolving member (17) consists of a revolving cylinder (27) supported on a hub (18) by means of radial blades (19) and having radial passage apertures (28) in a wall zone which adjoins each of the blades (19) in the opposite direction to the direction of revolution.

8. Apparatus according to claim 7, characterised in that the revolving member (17) is mounted on the fermenter (1) with a vertical axis and in that the foam inlet (13) consists of an axial bottom opening in the housing (15) extending into the fermenter (1) at the end face.

9. Apparatus for performing the method according to any one of claims 1 to 4, comprising a rotatably mounted revolving member (17) having radial blades (19) and axial passages between the blades (19), with a foam inlet (13) on one end face of the revolving member (17), said inlet (13) being connected to a fermenter (1), a gas outlet (20) on the other end face of the revolving member (17), and with a liquid outlet adjoining the revolving member (17), characterised in that the revolving member (17) consists of a revolving cylinder (27) which is supported on a hub (18) by means of the radial blades (19), and adjoining which the liquid outlet discharge line (21) taken out of the fermenter (1) extends by an inlet orifice (30) extending in the opposite direction to the direction of rotation of the revolving member (17).

10. Apparatus according to claim 9, characterised in that the revolving cylinder (27) projects axially beyond the blades (19) at least on one end face and in that the inlet orifice (30) of the liquid outlet discharge line (21) adjoins the inner wall of the portion (29) of the revolving cylinder (27) projecting beyond the blades (19).

11. Apparatus according to claim 9, characterised in that the outside of the revolving member (17) has at least one annular collecting channel (32) connected to the interior of the revolving cylinder (27) by passage apertures (33), the inlet orifice (30) of the liquid outlet discharge line (21) adjoining the inside of the outer wall of said channel.

12. Apparatus according to claim 11, characterised in that the annular collecting channel (33) is provided on the revolving cylinder (27) in the region of the gas outlet side.

13. Apparatus according to any one of claims 9 to 12, characterized in that the revolving member (17) is mounted with a vertical axis of rotation and its bottom open end face forms the foam inlet (13).

14. Apparatus according to claims 10 and 13, characterised in that the downwardly projecting part (29) of the revolving cylinder (27) forms a collecting channel (31) into which the discharge line (21) leads by its inlet orifice (30).

15. Apparatus according to any one of claims 9 to 14, characterised in that the discharge line (21) is taken out of the top of the fermenter (1).

## Revendications

1. Procédé de guidage de la mousse, dans un processus de fermentation s'opérant lors de la fabrication du vinaigre, la mousse qui se produit sur la surface d'un substrat de fermentation étant subdivisée, le long d'un trajet de transport, sous l'effet des forces centrifuges s'exerçant autour de l'axe d'un tronçon de transport, en donnant une proportion de gaz, à extraire en direction du tronçon de transport, et une proportion de liquide, se séparant radialement, présentant encore, le cas échéant, des restes de mousse,
caractérisé en ce que la proportion de liquide produite lors de la séparation du liquide est séparée pour être évacuée, avec les restes de mousse contenus, hors du processus de fermentation.

2. Procédé selon la revendication 1,
caractérisé en ce que les proportions de liquide et de mousse, séparées hors du processus de fermentation, sont retraitées indépendamment du produit fini obtenu à l'issu du processus de fermentation.

3. Procédé selon la revendication 1 ou 2,
caractérisé en ce que les proportions de liquide et de mousse séparées hors du processus de fermentation sont ajoutées par mélange au produit fini qui est issu du processus de fermentation.

4. Procédé selon la revendication 2 ou 3,
caractérisé en ce que les proportions de liquide et de mousse séparées hors du processus de fermentation sont l'objet d'un stockage intermédiaire avant retraitement.

5. Dispositif pour la mise en oeuvre du procédé selon l'une des revendications 1 à 4,
comprenant un corps de circulation (17) monté à rotation dans un carter (15), présentant des ailettes radiales (19) et, entre les ailettes (19), des passages axiaux, avec une admission de mousse (13) raccordée à un fermenteur (1), sur une première face frontale du carter (15), une extraction de gaz (20), sur l'autre face frontale du carter (15) et avec une évacuation de liquide dans la zone de l'enveloppe de carter,
caractérisé en ce que l'évacuation de liquide est reliée à une conduite d'évacuation (21) ressortant du fermenteur (1).

6. Dispositif selon la revendication 5,
caractérisé en ce que la conduite d'évacuation (21) est reliée à un récipient collecteur (22) isolé vis à vis du fermenteur (1).

7. Dispositif selon la revendication 5 ou 6,
caractérisé en ce que le corps de circulation (17) est composé d'un cylindre de circulation (27), soutenu sur un moyeu (18) à l'aide des ailettes radiales (19) et présentant des trous de passage (28) radiaux, dans une zone de paroi se raccordant chaque fois aux ailettes (19), dans la direction opposée à celle de la circulation.

8. Dispositif selon la revendication 7,
caractérisé en ce que le corps de circulation (17) est monté sur le fermenteur (1) avec un axe vertical et en ce que l'admission de mousse (13) est composée d'une ouverture de carter inférieure, axiale, ménagée dans le carter (15), qui pénètre frontalement dans le fermenteur (1).

9. Dispositif pour mettre en oeuvre le procédé selon l'une des revendications 1 à 4, comprenant un corps de circulation (17) monté à rotation, présentant des ailettes radiales (19) et, entre les ailettes (19), des passages axiaux, avec une admission de mousse (13) raccordée à un fermenteur (1), sur une première face frontale du carter (15), avec une extraction de gaz (20), sur l'autre face frontale du corps de circulation (17), et avec une évacuation de liquide se raccordant au corps de circulation (17), caractérisé en ce que le corps de circulation (17) est composé d'un cylindre de circulation (27), soutenu sur un moyeu (18) à l'aide des ailettes radiales (19) et auquel la conduite d'évacuation (21) d'évacuation de liquide, guidée hors du fermenteur (1), se raccorde, avec une embouchure d'admission (30) orientée dans le sens inverse du sens de rotation du corps de circulation (17).

10. Dispositif selon la revendication 9,
caractérisé en ce que le cylindre de circulation (27) dépasse axialement au-dessus des ailettes (19), au moins sur une face frontale,

11. Dispositif selon la revendication 9,
caractérisé en ce que le corps de circulation (17) présente en face extérieure au moins une goulotte collectrice (32) annulaire, reliée à l'espace intérieur du cylindre de circulation (27) au moyen d'ouvertures de passage (33) et sur la paroi extérieure de laquelle se raccorde, du côté intérieur, l'embouchure d'admission (30) de la conduite d'évacuation (21) de l'évacuation de liquide.

12. Dispositif selon la revendication 11,
caractérisé en ce que la goulotte collectrice annulaire (est prévue dans la zone de côté sortie des gaz sur le cylindre de circulation (27).

13. Dispositif selon l'une des revendications 9 à 12,
caractérisé en ce que le corps de circulation (17) est monté à rotation sur un axe de rotation vertical et forme, avec sa face frontale inférieure ouverte, l'admission de mousse (13).

14. Dispositif selon les revendications 10 et 13,
caractérisé en ce que le tronçon (29), faisant saillie vers le bas, du cylindre de circulation (27), constitue une goulotte collectrice (31), dans laquelle pénètre la conduite d'évacuation (21), par une embouchure d'admission (30).

15. Dispositif selon l'une des revendications 9 à 14,
caractérisé en ce que la conduite d'évacuation (21) est guidée vers le haut, hors du fermenteur (1).
